# EUROPEAN PATENT SPECIFICATION

(11) **EP 0 966 282 B1**
(45) Date of publication and mention of the grant of the patent: **25.06.2003**
(21) Application number: 98903447.5
(22) Date of filing: 07.01.1998
(51) Int. Cl.: A61K 31/41, A61K 31/415, A61K 31/44, A61K 31/47, A61K 31/505, A61K 38/04, A61P 9/00, A61P 9/04

(54) **USE OF THE ANGIOTENSIN II ANTAGONISTS LOSARTAN AND EXP-3174 TO TREAT SYMPTOMATIC HEART FAILURE**
VERWENDUNG DER ANGIOTENSIN II-ANTAGONISTEN LOSARTAN UND EXP-3174 ZUR BEHANDLUNG VON SYMPTOMATISCHES HERZVERSAGEN
UTILISATION DES ANTAGONISTES D'ANGIOTENSINE II LOSRTAN ET EXP-3174 DANS LE TRAITEMENT D'UNE INSUFFISANCE CARDIAQUE SYMPTOMATIQUE

(30) Priority: 10.01.1997 US 34927 P; 28.02.1997 GB 9704197
(43) Date of publication of application: 29.12.1999
(73) Proprietor: MERCK & CO., INC., Rahway, New Jersey 07065 (US)
(72) Inventor: BEERE, Polly, A., Rahway, NJ 07065 (US); CHANG, Paul, I., Rahway, NJ 07065 (US); PITT, Bertram, Rahway, NJ 07065 (US); RUCINSKA, Ewa, J., Rahway, NJ 07065 (US); SEGAL, Robert, Rahway, NJ 07065 (US); SHARMA, Divakar, Rahway, NJ 07065 (US); SNAVELY, Duane, B., Rahway, NJ 07065 (US)
(74) Representative: Hiscock, Ian James
(86) International application number: US9800534
(87) International publication number: WO98030216

(56) References cited:
- WO-A-96/40257
- O. LENZ ET AL.: "Effects of angiotensin II and angiotensin-converting enzyme inhibitors on human myocardium." EUR. J. PHARMACOL., vol. 294, no. 1, 1995, pages 17-27, XP002065907
- N.A. AWAN ET AL.: "Direct selective blockade of the vascular angiotensin II receptors in therapy for hypertension and severe congestive heart failure." AM. HEART JOURNAL, vol. 131, no. 1, 1996, pages 177-185, XP002065908
- "candesartan cilexetil" DRUGS OF THE FUTURE, vol. 21, no. 7, 1996, pages 738-739, XP002065909
- "candesartan cilexetil" DRUGS OF THE FUTURE, vol. 20, no. 7, 1995, pages 740-742, XP002065910
- T. LEJEMTEL ET AL.: "Irbesartan-a new angiotensin II antagonist: acute hemodynamic effects in patients with heart failure." CIRCULATION, vol. 94, no. 8 suppl., 1996, pages I622-I623, XP002065911
- I. CROZIER ET AL.: "Losartan in heart failure" CIRCULATION , vol. 91, no. 3, 1995, pages 691-697, XP002065912
- A. FUJIMORI ET AL.: "Effect of chronic treatment with YM358, an angiotensin II receptor (AT1) antagonist , in experimental heart failure." JPN. J. PHARMACOL., vol. 67, no. suppl. 1, 1995, page 124P XP002065913
- T. KUSAYAMA ET AL.: "Effect ofYM358, an angiotensin II receptor(AT1) antagost, on anesthetized dogs in acute left ventricular failure." JPN. J. PHARMACOL., vol. 67, no. suppl.1, 1995, page 124P XP002065914
- Y. INADA ET AL.: "nonpeptide angiotensin II- receptor antagonists." FOLIA HARMACOL. JPN., vol. 104, no. 3, 1994, pages 217-228, XP002065915
- P. BÜHLMAYER ET AL.: "Valsartan, a potent, orally acie angiotensin II antagonist developed from the structurally new amino acid series." BIOORG. MED. CHEM. LETT., vol. 4, no. 1, 1994, pages 29-34, XP002065916
- B. NO L ET AL: "Clinical and hormonal effects of the new angiotensin II receptor antagonist LRB081" J. CARDIOVASC. PHARMACOL., vol. 28, no. 2, 1996, pages 252-258, XP002065917

## Description

### BACKGROUND OF THE INVENTION

Angiotensin-converting-enzyme. (ACE) inhibitors have been shown to reduce morbidity and mortality in patients with chronic heart failure and systolic left ventricular dysfunction as well as in patients post myocardial infarction. (See The CONSENSUS Trial Study Group. Effects of enalapril on mortality in severe congestive heart failure. Results of the Cooperative North Scandinavian Enalapril Survival Study (CONSENSUS). N Engl J Med 1987; 316: 1429-1435; The SOLVD Investigators. Effect of enalapril on survival in patients with reduced left ventricular ejection fractions and congestive heart failure. N Engl J Med 1991; 325: 293-302; The SOLVD Investigators. Effect of enalapril on mortality and the development of heart failure in asymptomatic patients with reduced left ventricular ejection fractions. N Engl J Med 1992; 327: 685-691; Cohn JN, Johnson G, Ziesche S, et al. A comparison of enalapril with hydralazine-isosorbide dinitrate in the treatment of chronic congestive heart failure. N Engl J Med 1991; 325: 303-310; Pfeffer MA, Braunwald E, Moye LA, et al. on behalf of the SAVE Investigators. Effect of captopril on mortality and morbidity in patients with left ventricular dysfunction after myocardial infarction. Results of the Survival and Ventricular Enlargement Trial. N Engl J Med 1992; 327: 669-677; The Acute Infarction Ramipril Efficacy (AIRE) Study Investigators. Effect of ramipril on mortality and morbidity of survivors of acute myocardial infarction with clinical evidence of heart failure. Lancet 1993; 342: 812-828; Fonarow GC, Chelimsky-Fallick C, Warner Stevenson L, et al. Effect of direct vasodilation with hydralazine versus angiotensin-converting enzyme inhibition with captopril on mortality in advanced heart failure: the Hy-C trial. J Am Coll Cardiol 1992; 19: 842-850; Gruppo Italiano per lo Studio delia Sopravvivenza nell'infarto Miocardico. GISSI-3: Effects of lisinopril and transdermal glyceryl trinitrate singly and together on 6-week mortality and ventricular function after acute myocardial infarction. Lancet 1994; 343: 1115-1122; ISIS Collaborative Group OU. ISIS-4: Randomized study of oral isosorbide mononitrate in over 50,000 patients with suspected acute myocardial infarction. Circulation 1993; 88: I394.) The benefits of ACE inhibitors have been attributed to blockade of angiotensin II production and/or to a decrease in the breakdown of bradykinin. (See Pitt B, Chang P, Timmermans P. Angiotensin II receptor antagonists in heart failure: Rationale and design of the Evaluation of Losartan in the Elderly (ELITE) Trial. Cardiovascular Drugs and Therapy 1995; 9: 693-700; and Gavras I. Bradykinin-mediated effects of ACE inhibition. Kidney Int 1992; 42: 1020-1029.) Bradykinin has been shown to have beneficial effects associated with the release of nitric oxide and prostacyclin which may contribute to the hemodynamic effects of ACE inhibition. Bradykinin may, however, also be responsible for certain adverse effects associated with use of ACE inhibitors, such as cough, angioedema, renal dysfunction, and hypotension. (See Pitt B, Chang P, Timmermans P. Angiotensin II receptor antagonists in heart failure: Rationale and design of the Evaluation of Losartan in the Elderly (ELITE) Trial. Cardiovascular Drugs and Therapy 1995; 9: 693-700; Gavras I. Bradykinin-mediated effects of ACE inhibition. Kidney Int 1992; 42: 1020-1029; Israili ZH, Hall WD. Cough and angioneurotic edema associated with angiotensin-converting enzyme inhibitor therapy. A review of the literature and pathophysiology. Ann Intern Med 1992; 117: 234-242; Chalmers D, Dombey SL, Lawson DH. Post-marketing surveillance of captopril (for hypertension): a preliminary report. Br J Clin Pharmacol 1987; 24: 343-349; Lacourciere Y, Brunner H, Irwin R, et al. and the Losartan Cough Study Group. Effects of modulators of the renin-angiotensin-aldosterone system on cough. J Hypertension 1994; 12: 1387-1393.) These adverse effects may account in part for the fact that ACE inhibitors are used in less than 30 percent of patients with heart failure in spite of their proven clinical benefit. (See Stafford RS, Saglam D, Blumenthal D. Low rates of angiotensin-converting enzyme inhibitor use in congestive heart failure. Circulation 1996; 94: I-194(Abstract)).

The development of orally-active, nonpeptidic angiotensin II type 1 receptor antagonists such as losartan, has provided the opportunity to block the angiotensin II type 1 receptor specifically without increasing bradykinin levels. (See Timmermans P, Wong PC, Chiu AT, et al. Angiotensin II receptors and angiotensin II receptor antagonists. Pharmacol Reviews 1993; 45: 205-251.) Since angiotensin II may be produced by alternate pathways, losartan may offer additional advantages over treatment with ACE inhibitors where blockade of the effects of angiotensin II may be incomplete. (See Miura S, Ideishi M, Sakai T, et al. Angiotensin II formation by an alternative pathway during exercise in humans. J Hypertension 1994; 12: 1177-1181; Urata H, Kinoshita A, Misono KS, Bumpus FM, Husain A. Identification of a highly specific chymase as the major angiotensin II-forming enzyme in the human chymase. J Biol Chem 1990; 265: 22348-22357; Urata H, Strobel F, Ganten D. Widespread tissue distribution of human chymase. J Hypertension 1994; 12: S17-S22; Aldigier JC, Huang H, Dalmay F, et al. Angiotensin-converting enzyme inhibition does not suppress plasma angiotensin II increase during exercise in humans. J Cardiovasc Pharmacol 1993; 21: 289-295.) Losartan is indicated for the treatment of hypertension in many countries and in earlier studies in patients with symptomatic heart failure, oral losartan produced beneficial hemodynamic effects both acutely and with chronic dosing. (See Crozier I, Ikram H, Awan N, et al. Losartan in heart failure: Hemodynamic effects and tolerability. Circulation 1995; 91: 691-697; and Gottlieb SS, Dickstein K, Fleck E, et al. Hemodynamic and neurohormonal effects of the angiotensin II antagonist losartan in patients with congestive heart failure. Circulation 1993; 88: 1602-1609.)

The Evaluation of Losartan In The Elderly (ELITE) Study was conducted to compare effects on renal function, morbidity/mortality, and tolerability of long-term treatment with losartan versus captopril, in older patients with symptomatic heart failure.

Additional prior art includes "Candesartan Cilextil," *Drugs of the Future,* vol. 21, no.7, 1996, pages 738-739, which discloses the use of candesartan cilextil for the treatment of heart failure, and as an effective treatment for myocarditis and chronic heart failure. PCT International Publication WO-A-96/40257 discloses biphenylmethylbenzimidazoles as angiotensin II antagonists useful for the treatment of hypertension and congestive heart failure. N.A. Awan *et al., American Heart Journal,* vol. 131, no.1, 1996, pages 177-185 discloses losartan as useful for treating hypertension and for improving the hemodynamics typical of chronic heart failure. I. Crozier *et al., Circulation* vol. 91, no.3, 1995, pages 691-697 discloses the treatment with losartan improved the hemodynamics in symptomatic heart failure patients. T. Lejemtel *et al., Circulation* vol. 94, no.8 suppl., 1996, page 1622 discloses use of irbesartan for chronic therapy in heart failure. A. Fujimori *et al., Japanese Journal of Pharmacology,* vol. 67, no.suppl.1, 1995, page 124P discloses the use of YM358 for the treatment of symptomatic heart failure. O. Lenz, *et al., European Journal of Pharmacology,* vol. 294, no. 1, 1995, page 17-27 discloses that AT1 receptors exclusively mediate the direct positive inotropic effects in atrial myocardium. PCT International Publication WO-A-97/49394 discloses solid oral dosage form of valsartan for use in the treatment of hypertension and congestive heart failure and a solid oral dosage form of and valsartan in combination with hydrochlorothiazide for use in the treatment of hypertension.

### SUMMARY OF THE INVENTION

The present invention relates to the use of an angiotensin II (AT₁) receptor antagonist selected from losartan, EXP-3174, or a pharmaceutically acceptable salt thereof, for the manufacture of a medicament for reducing mortality of a symptomatic heart failure patient.

The invention also relates to the use of an angiotensin II (AT₁) receptor antagonist selected from losartan, EXP-3174, or a pharmaceutically acceptable salt thereof, for the manufacture of a medicament for (a) reducing sudden cardiac death of a symptomatic heart patient; (b) reducing mortality and sudden cardiac death of a symptomatic heart failure patient; (c) preventing sudden cardiac death of a symptomatic heart failure patient; or (d) reducing hospitalization of a symptomatic heart failure patient.

### BRIEF DESCRIPTION OF THE FIGURES

Figure 1. ELITE Study Profile.
   (* While on study therapy; and ** Withdrawn from assigned therapy, but still followed up for the intention-to-treat analysis of secondary endpoint.)
Figure 2. Kaplan-Meier Survival Curves among Patients with Chronic Heart Failure in the Losartan and Captopril Groups.
   (Patients in the losartan group had a 46 percent lower risk of death (RR= risk reduction) than patients in the captopril group (P=0.035). Patients were followed for 48 weeks.)
Figure 3. Effect of Losartan on Death in Various Subgroups.
   (⁼ One losartan-treated patient did not have EF measured; and ⁼⁼ Based on patient history. For each subgroup, the percentage of risk reduction (RR) with losartan is plotted (solid squares). Horizontal lines represent 95 percent confidence intervals. The size of each square is proportional to the percentage of events in the subgroup. The light square at the bottom of the panel represents the overall result for death. The bold vertical line corresponds to a finding of no effect. The RRs in individual subgroups are generally consistent with the overall RR, except for females. Interaction tests for the effect of losartan on mortality in gender was significant at the 10 percent level (p=0.053). Tests for interaction in the other subgroups were not significant. NYHA denotes New York Heart Association.)

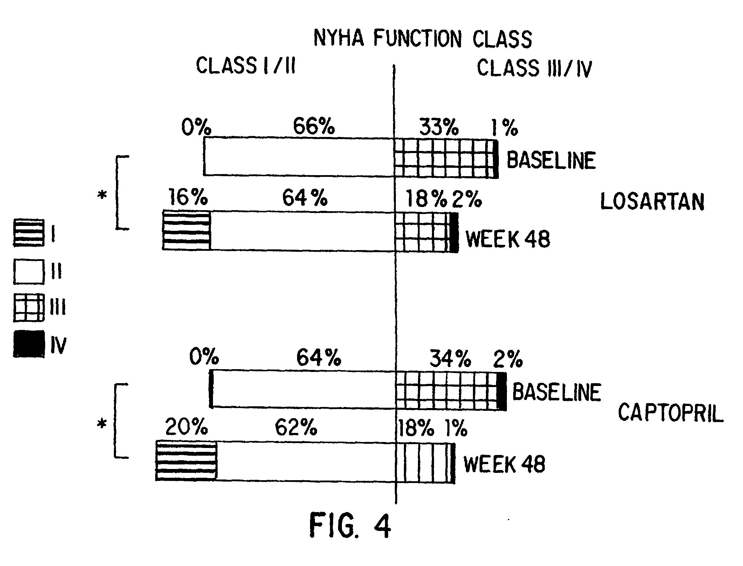

Figure 4. Changes in New York Heart Association Functional Class. (*P≤0.001. Percent of patients with change in New York Heart Association (NYHA) Functional Class at week 48 versus Baseline.)

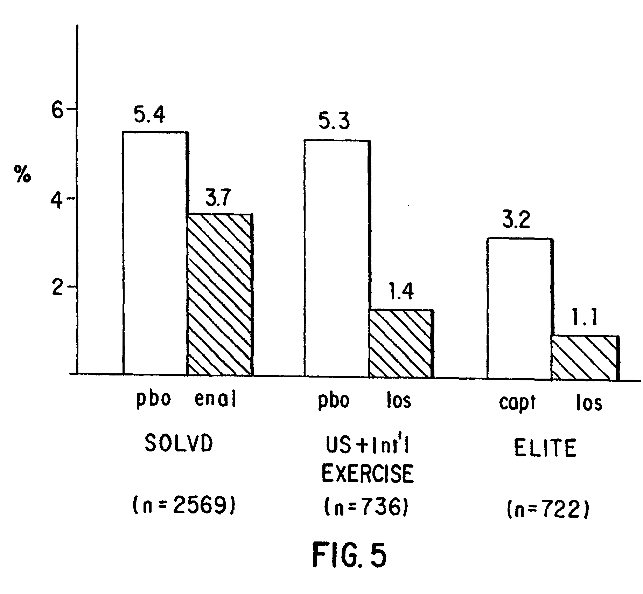

Figure 5. Comparison of twelve-week mortality data from the SOLVD study, the losartan exercise studies (U.S. and international combined), and the ELITE study. (pbo = placebo; enal = enalapril; los = losartan; capt = captopril).

### DETAILED DESCRIPTION OF THE INVENTION

This invention concerns the use of an angiotensin II (AT₁) receptor antagonist selected from losartan, EXP-3174, or a pharmaceutically acceptable salt thereof, for the manufacture of a medicament for reducing mortality of a symptomatic heart failure patient.

The angiotensin II receptor antagonists of use in the present invention are:
2-Butyl-4-chloro-1-[(2'-tetrazol-5-yl)biphenyl-4-yl]methyl]-5-(hydroxymethyl)imidazole (also known as losartan); and
2-Butyl-4-chloro-1-[(2'-tetrazol-5-yl)biphenyl-4-yl]methylimidazole-5-carboxylic acid (also known as EXP-3174) or its pharmaceutically acceptable salt thereof. The most preferred embodiment is the use, as recited above, wherein the symptomatic heart failure patient is age 65 or older.

This invention also relates to the use of an angiotensin II (AT₁) receptor antagonist selected from losartan, EXP-3174, or a pharmaceutically acceptable salt thereof, for the manufacture of a medicament for (a) reducing sudden cardiac death of a symptomatic heart patient; (b) reducing mortality and sudden cardiac death of a symptomatic heart failure patient; (c) preventing sudden death of a symptomatic heart failure patient; or (d) reducing hospitalization of a symptomatic heart failure patient.

In a preferred embodiment of this aspect of the present invention, the symptomatic heart failure patient is 65 years of age or older.

### EXAMPLE 1

### ELITE Study

### Patients and methods

The ELITE studywas a prospective double-blind, randomized, parallel, captopril-controlled clinical trial conducted at 125 centers in the United States, Europe and South America. The study was approved by institutional review boards at each site; all patients gave written informed consent. An independent Data and Safety Monitoring Committee monitored the progress of the study. See Pitt B, Chang P, Timmermans P. Angiotensin II receptor antagonists in heart failure: Rationale and design of the Evaluation of Losartan in the Elderly (ELITE) Trial. Cardiovascular Drugs and Therapy 1995; 9: 693-700.

### Patient Population

Patients were 65 years or older (two thirds 70 years or older) with symptomatic heart failure (New York Heart Association Class II-IV), decreased left ventricular ejection fraction of 40 percent or less, and had no history of prior ACE inhibitor therapy. A detailed description of enrollment and exclusion criteria and study design has been presented previously. See Pitt B, Chang P, Timmermans P. Angiotensin II receptor antagonists in heart failure: Rationale and design of the Evaluation of Losartan in the Elderly (ELITE) Trial. Cardiovascular Drugs and Therapy 1995; 9: 693-700.

### Randomization and Study Therapy

Following a two-week placebo run-in, patients were randomized to 48 weeks of active therapy, either to captopril 6.25 mg titrated to 12.5 mg, 25 mg and then 50 mg three times daily (plus placebo for losartan) or to losartan 12.5 mg, titrated to 25 mg and then 50 mg once daily (plus placebo for captopril). Titration generally occurred at 7-day intervals as tolerated. Treatment with all other concomitant cardiovascular therapies was permitted with the exception of open-label ACE inhibitors. Randomized patients were stratified by age (less than 70 and 70 years or greater).

### Evaluation of Patients

Clinical assessments were performed weekly during titration and at three-month intervals thereafter. Laboratory evaluations were performed at weeks three, six, and twelve, and at three month intervals thereafter.

### Study Endpoints

The primary endpoint of the study was a safety measure of the development of renal dysfunction, defined as an increase in serum creatinine ≥0.3 mg/dL from baseline that was confirmed by a repeat measurement in 5 to 14 days of the first determination, during continued treatment. All-cause mortality and heart failure hospitalizations were each prespecified endpoints, and the composite of death and/or heart failure hospitalizations was added as the secondary endpoint by protocol amendment based on data from two placebo-controlled 12 week exercise studies (of approximately 350 patients each), demonstrating a beneficial effect on this endpoint. See Example 2) Hypotension-related symptoms, clinically important serum potassium increases (≥ 0.5 mEq/L), and cough, all originally secondary endpoints, were moved to tertiary endpoints. All deaths (including cause of death) and hospitalizations were adjudicated by an independent Clinical Endpoint Adjudication Committee, blinded to study treatment (see *panel* for mortality classification). Other prospectively-defined measures included myocardial infarction/hospitalization for unstable angina, worsening of heart failure, New York Heart Association functional classification, discontinuation from the study due to study drug intolerance, changes in neurohormonal profile.

### Panel: Mortality Classification

- Sudden Cardiac Death: Death occurring without warning or within 1 hour of symptoms
- Death due to Progressive Heart Failure: Death preceded by worsening signs and/or symptoms of heart failure, including cardiogenic shock
- Fatal Myocardial Infarction: Death associated with autopsy-verified myocardial infarction or death within 28 days of a hospital-verified acute myocardial infarction provided no other cardiac or non-cardiac cause of death is found
- Death due to Other Cardiac Causes: Death due to other cardiac causes, such as arrhythmia
- Death due to Other Vascular Causes: Death due to vascular events such as stroke, pulmonary embolus, ruptured aneurysm, etc.
- Death due to non-cardiovascular causes: Death not due to any cardiac or vascular events

### Statistical Methods

The power and sample-size estimates for this study have been described. See Pitt B, Chang P, Timmermans P. Angiotensin II receptor antagonists in heart failure: Rationale and design of the Evaluation of Losartan in the Elderly (ELITE) Trial. Cardiovascular Drugs and Therapy 1995; 9: 693-700. Analysis of renal dysfunction (increases in serum creatinine) was based upon a modified intent-to-treat population; i.e., all patients were analyzed according to their randomization group, and an endpoint was declared only if initial and confirmatory elevations occurred while on double-blind therapy. Patients who discontinued from the study without meeting this endpoint were censored in the time-to-event analysis at the time of study discontinuation.

Analyses of deaths and heart failure hospitalizations (adjudicated endpoints) were based on an intent-to-treat population; all patients discontinued prematurely were followed through the specified 48-week period of the study. Patients not meeting the endpoint were censored in the time-to-event analysis either at the time of study completion (for patients who completed) or at the end of the 48-week follow-up period (for patients who discontinued).

For all time-to-event data, survival analyses were based upon the log-rank test. The effect of treatment group in the model was tested controlling for the stratification factor (age category [less than 70 or 70 years and older]). The time to *first* event was used for each endpoint. Risk reductions were based upon Mantel-Haenszel adjusted (for age catagory) relative risk estimates.

The primary endpoint was tested at the 5% level of significance. No multiplicity adjustments were made for the test of the secondary endpoint or for other tests of interest; unadjusted p-values are presented.

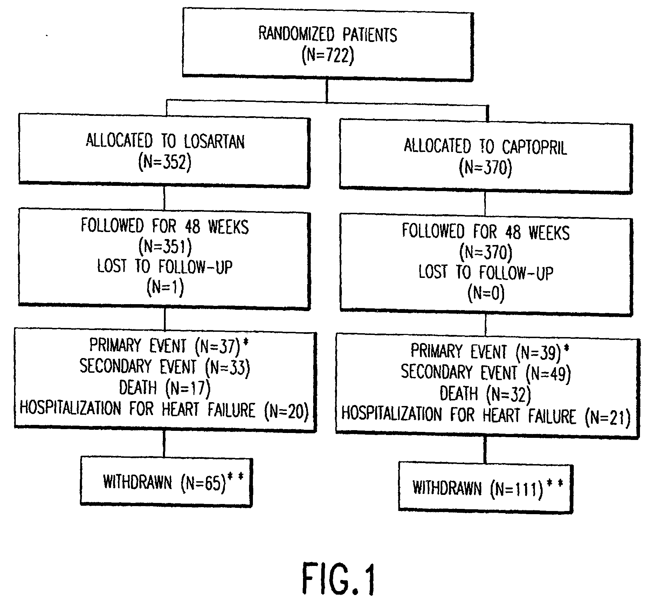

Over approximately a two-year recruitment period, 722 patients enrolled, 352 were randomized to losartan and 370 to captopril (Figure 1). The two treatment groups were similar with respect to all baseline characteristics (Table 1). Concomitant therapies during the study were similar between the two treatment groups; diuretics were used in over 70 percent of patients, digitalis in over 55% of patients and non-ACE inhibitor vasodilatory drugs (including hydralazine and nitrates) in over 50 percent of patients in both treatment groups. Three hundred patients (85 percent) were titrated to the target dose of losartan 50mg once daily, and 321 patients (84 percent) in the captopril group were titrated to the target dose of 50mg three times a day. Seventy-five percent of losartan-treated patients remained on the targeted dose of 50mg daily (mean daily dose 42.6 mg per day), and 71 percent of captopril patients remained on the targeted dose of 50mg three times a day (mean daily dose 122.7 mg per day).

**Table 1.**

| Baseline Clinical Characteristics and Drug Therapy, According to Treatment Group | | |
|---|---|---|
| CHARACTERISTIC | LOSARTAN (n=352) | CAPTOPRIL (n=370) |
| | *number (percent) of patients* | |
| Sex | | |
| Male | 234 (66.5) | 248 (67.0) |
| Female | 118 (33.5) | 122 (33.0) |
| Age (years) | | |
| <70 | 95 (27.0) | 119 (32.2) |
| ≥70 | 257 (73.0) | 251 (67.8) |
| Mean (S.D.) | 74 ( 5.8) | 73 ( 6.1) |
| Race | | |
| Caucasian | 320 (90.9) | 326 (88.1) |
| Black | 16 ( 4.6) | 18 (4.9) |
| Other | 16 ( 4.6) | 26 ( 7.0) |
| Etiology of Heart Failure^{†} | | |
| Ischemic Heart Disease | 242 (68.8) | 250 (67.6) |
| Non-Ischemic Heart Disease | 110 (31.3) | 120 (32.4) |
| NYHA Functional Class^{††} | | |
| II | 231 (65.6) | 237 (64.1) |
| III | 116 (33.0) | 126 (34.1) |
| IV | 5 ( 1.4) | 7 ( 1.9) |
| Drug Therapy | | |
| Diuretics | 260 (73.9) | 275 (74.3) |
| Digitalis | 199 (56.5) | 209 (56.5) |
| Hydralazine | 12 (3.4) | 12 (3.2) |
| Nitrates | 180 (51.1) | 191 (51.6) |
| Calcium Channel Blockers | 123 (34.9) | 121 (32.7) |
| Potassium Supplement | 91 (25.9) | 89 (24.1) |
| Anticoagulants | 60 (17.0) | 69 (18.6) |
| Beta-Blockers | 55 (15.6) | 63 (17.0) |
| Antiarrhythmics | 37 (10.5) | 39 (10.5) |
| Secondary Diagnoses^{†} | | |
| Myocardial Infarction | 184 (52.3) | 177 (47.8) |
| Hypertension | 201 (57.1) | 212 (57.3) |
| Atrial Fibrillation | 86 (24.4) | 82 (22.2) |
| Diabetes mellitus | 94 (26.7) | 89 (24.1) |
| Renal Insufficiency | 21 ( 6.0) | 26 ( 7.0) |
| Stroke | 32 ( 9.1) | 37 (10.0) |
| Current smoker (cigarettes) | 39 (11.1) | 45 (12.2) |

| | *mean (S.D.)* | |
|---|---|---|
| Ejection Fraction (%) | 31 ( 7.2) | 30 ( 7.6) |
| Serum Creatinine (mg/dl)^{†††} | 1.2 ( 0.4) | 1.2 ( 0.4) |
| Serum Potassium (mEq/L) ^{†††} | 4.3 ( 0.4) | 4.3 ( 0.5) |
| Heart Rate (beats per minute) | 73 (11.7) | 74 (10.4) |
| Blood Pressure | | |
| Systolic (mmHg) | 137 (17.6) | 137 (19.1) |
| Diastolic (mmHg) | 79 ( 9.4) | 79 (10.6) |
| Weight (kg) | 76 (33.1) | 74 (33.8) |

| | | |
|---|---|---|
| *Key to Table 1:* ^{†} Based on patient history. | | |
| ^{††} NYHA denotes New York Heart Association. | | |
| ^{†††}To convert to micromoles per liter, multiply by 88.4; mEq/L is the equivalent of millimoles per liter. | | |

### Renal Dysfunction

There was no significant difference between losartan and captopril in terms of persistent increases (i.e., confirmed by a repeat measurement during continued treatment) in serum creatinine ≥0.3 mg/dL from baseline (10.5 percent versus 10.5 percent, p=0.629; Table 2). Single rises of serum creatinine ≥0.3 mg/dL were documented in 92 losartan-treated patients (26.1 percent) and 110 captopril-treated patients (29.7 percent), with a trend towards fewer events occuring in losartan-treated patients (14 percent risk reduction, 95 percent confidence intervals -0.09 to 0.32; p=0.060). Of these patients with single rises of serum creatinine ≥0.3 mg/dL, 68 percent had confirmation measurements performed while on active therapy per protocol; 55 percent of the patients who had these confirmation measurements met the endpoint.

### Death and/or Heart Failure Hospitalizations

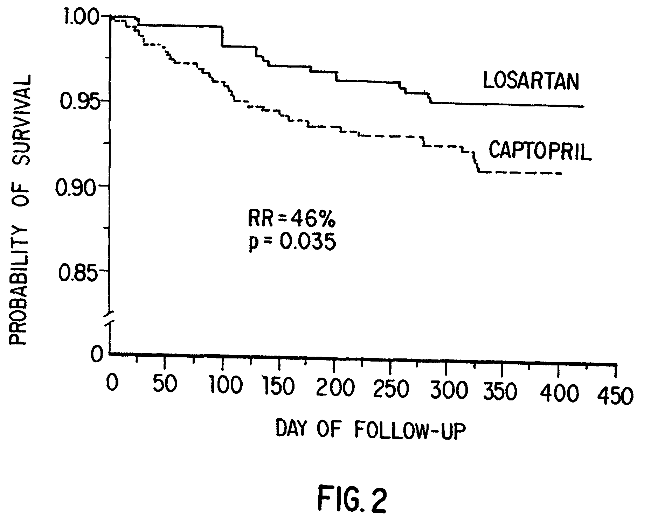

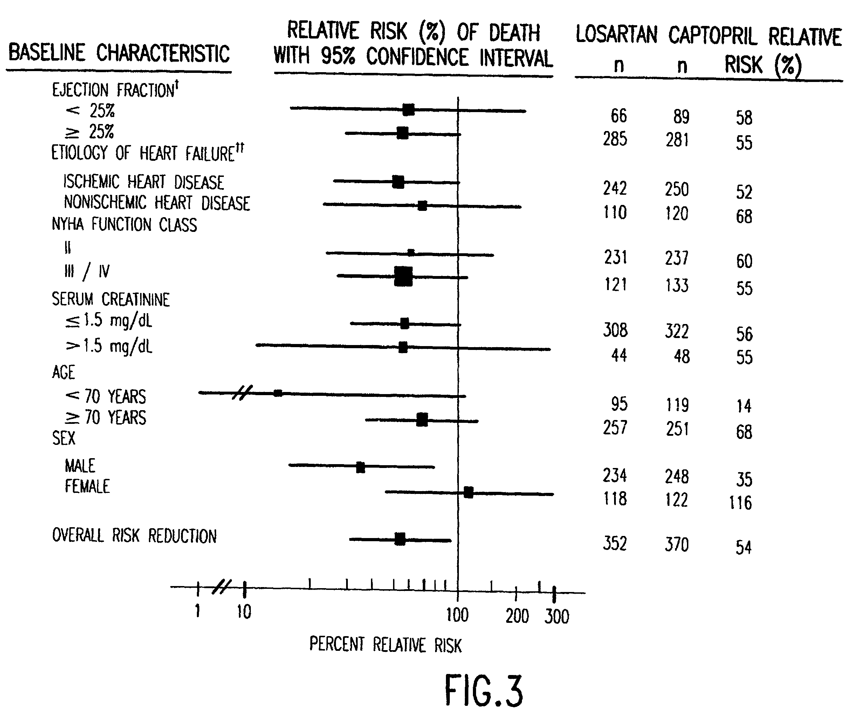

Follow-up data on death and hospitalizations were complete except for one losartan-treated patient who was discontinued after one dose of study medication. During the course of the study, death and/or heart failure hospitalizations occurred in 33 of 352 losartan-treated patients (9.4 percent) compared to 49 of 370 captopril-treated patients (13.2 percent) (p=0.075). This decrease in death and/or heart failure hospitalizations observed with losartan versus captopril was entirely due to a 46 percent decrease in total mortality (17 versus 32 patients [p=0.035]; Table 3). The cumulative survival curves (intent-to-treat) separated early and remained so throughout the 48-week study period (Figure 2). The observed decrease in total mortality was primarily due to a reduction in sudden cardiac death (five versus 14 patients, p=0.043); only one patient died of progressive heart failure in each treatment group (Table 3). Fatal myocardial infarction occurred in one losartan-treated patient versus four in the captopril arm. The effect of losartan versus captopril on mortality was generally a consistent observation across the different subgroups (Figure 3), except in a subpopulation of female patients (9 of 118 losartan-treated versus 8 of 122 captopril-treated female patients died).

Fewer losartan-treated patients were hospitalized overall for any reason during the 48-week study observation period than captopril-treated patients (86 [24.4 percent] versus 119 [32.2 percent]; p=0.018). The incidence of heart failure hospitalizations however was not different between treatment groups (both 5.7 percent) (Table 3).

### Functional Status and Norepinephrine Levels

New York Heart Association functional class improved similarly with losartan and captopril treatment (p≤0.001 versus baseline for each treatment group); 80 percent of losartan-treated patients and 82 percent of captopril-treated patients were classified as New York Heart Association Class I or II at the end of the study versus 66 percent of losartan-treated patients and 64 percent of captopril-treated patients at baseline (Figure 4). With regard to plasma norepinephrine levels, a three percent decrease from a geometric mean baseline of 469 pg/mL was observed at 48 weeks with losartan treatment, as compared to a five percent increase from a geometric mean baseline of 424 pg/mL with captopril (p = NS between treatment groups).

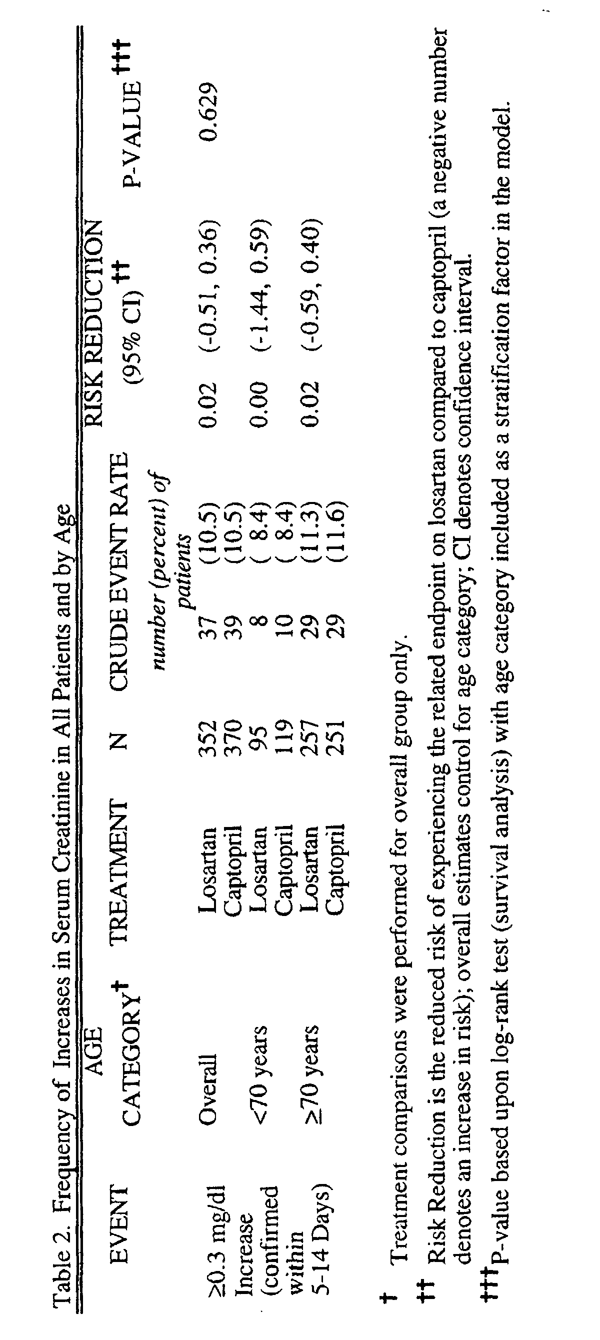

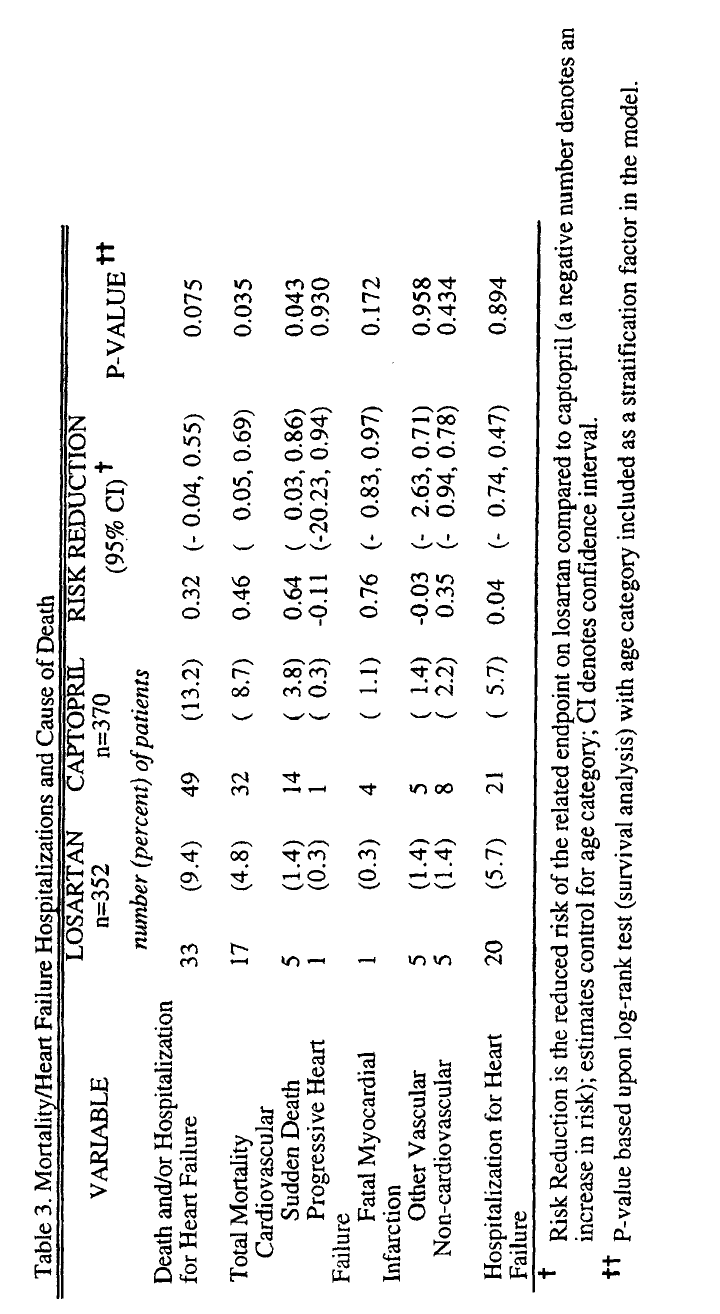

ELITE is the first long-term (48 weeks) study to compare the effect of treatment with losartan, an angiotensin II type 1 receptor antagonist, to an ACE inhibitor (captopril) in patients with symptomatic heart failure and systolic left ventricular dysfunction. Captopril was chosen as the comparative ACE inhibitor in this study because it had been suggested to have less adverse renal effects than longer-acting ACE inhibitors. (See Packer, M., Lee, W.H., Yushak, M., Medina, N.: Comparison of Captopril and Enalapril in patients with severe chronic heart failure. N Engl J Med; 315: 847-853.) The incidence of persistent renal dysfunction, as defined by a persisting increase in serum creatinine of 0.3 mg/dL (the primary endpoint), was not different between losartan- and captopril-treated patients (both 10.5%). The observation overall that both losartan and captopril were relatively well tolerated with regard to renal function was evident, given that less than 2% of patients discontinued for this reason in either group. This is of clinical relevance for treating older patients with heart failure.

The results of this study demonstrate that treatment with losartan resulted in a 46% reduction in all-cause mortality compared to captopril, a drug with demonstrated survival benefit in several studies. (See Pfeffer MA, Braunwald E, Moye LA, et al. on behalf of the SAVE Investigators. Effect of captopril on mortality and morbidity in patients with left ventricular dysfunction after myocardial infarction. Results of the Survival and Ventricular Enlargement Trial. N Engl J Med 1992; 327: 669-677; Fonarow GC, Chelimsky-Fallick C, Warner Stevenson L, et al. Effect of direct vasodilation with hydralazine versus angiotensin-converting enzyme inhibition with captopril on mortality in advanced heart failure: the Hy-C trial. J Am Coll Cardiol 1992; 19: 842-850; and ISIS Collaborative Group OU. ISIS-4: Randomized study of oral isosorbide mononitrate in over 50,000 patients with suspected acute myocardial infarction. Circulation 1993; 88: I394.) The cumulative survival benefit of losartan was observed early during the study, persisted throughout the 48-week treatment period, and was consistent among all subgroups except in female patients. It should be noted that the number of female patients enrolled in this study was relatively small; and the mortality benefit could not be demonstrated in this study. The greater drop-out rate in the captopril-treated patients did not account for the beneficial effects of losartan on total mortality; the treatment difference in total mortality was primarily due to those who remained on active therapy.

An improvement in survival with losartan of similar magnitude has also been observed in two placebo-controlled 12-week exercise studies. See Klinger G, Jaramillo N, Ikram H, et al. Effects of losartan on exercise capacity, morbidity and mortality in patients with symptomatic heart failure. J Am Coll Cardiol 1996 (in press); (Abstract). The three-month control group mortality rates in the exercise studies and the present study were comparable to the placebo and enalapril mortality rates respectively in the Studies of Left Ventricular Dysfunction (SOLVD) trial (Figure 5). See The SOLVD Investigators. Effect of enalapril on survival in patients with reduced left ventricular ejection fractions and congestive heart failure. N Engl J Med 1991; 325: 293-302. Despite the limitations of cross-study comparisons, treatment with losartan in the exercise studies and the present study was associated with comparably low mortality rates which were less than the observed mortality rates for both placebo and enalapril in SOLVD.

Prior to the use of ACE inhibitors, progressive heart failure accounted for approximately 50% of deaths due to heart failure. See Applefield MM. Chronic congestive heart failure: Where have we been? Where are we heading? Am J Med 1986; 80: 73-77. In patients with mild to moderate heart failure treated with an ACE inhibitor, death due to progressive heart failure has diminished so that sudden cardiac death is currently the predominant mode of death. For example, in the recently discontinued Survival With Oral *d*-Sotalol (SWORD) trial, in which patients with mild to moderate heart failure or left ventricular dysfunction were randomized to d-Sotolol or placebo on a background of usual therapy including an ACE inhibitor, arrhythmic death accounted for approximately two-thirds of total mortality in the placebo group, while progressive heart failure for only one-sixth. See Waldo AL, Camm AJ, deRuyter H, et al. for the SWORD Investigators. Effect of d-sotalol on mortality in patients with left ventricular dysfunction after recent and remote myocardial infarction. Lancet 1996; 348: 7-12. In the present study, sudden cardiac death was also the most prominent cause of death in the captopril-treated patients. Of interest, the observed reduction in mortality on losartan compared to captopril-treated patients in the present study was primarily due to a reduction in sudden cardiac death (see Table 3). Few patients died due to progressive heart failure or to fatal myocardial infarction in either treatment group, although numerically, fatal myocardial infarction deaths occurred less with losartan.

The mechanism by which losartan reduces sudden cardiac death in comparison to captopril is uncertain. Whether the reduction in sudden cardiac death was due to more complete blockade of angiotensin II effects or an intrinsic antiarrhythmic/antifibrillatory property of losartan remains to be determined. ACE activity may not be completely suppressed by the presently studied captopril dosing regimen (target dose of 50 mg three times daily), which is a regimen considered to have a mortality benefit. See Cohn JN. The management of chronic heart failure. N Engl J Med 1996; 335: 490-498. In addition angiotensin II may also be formed by non-ACE-dependent pathways. (See Miura S, Ideishi M, Sakai T, et al. Angiotensin II formation by an alternative pathway during exercise in humans. J Hypertension 1994; 12: 1177-1181; Urata H, Kinoshita A, Misono KS, Bumpus FM, Husain A. Identification of a highly specific chymase as the major angiotensin II-forming enzyme in the human chymase. J Biol Chem 1990; 265: 22348-22357; Urata H, Strobel F, Ganten D. Widespread tissue distribution of human chymase. J Hypertension 1994; 12: S17-S22; and Aldigier JC, Huang H, Dalmay F, et al. Angiotensin-converting enzyme inhibition does not suppress plasma angiotensin II increase during exercise in humans. J Cardiovasc Pharmacol 1993; 21: 289-295.) It is possible that more complete blockade of angiotensin II effects by losartan may result in more complete suppression of catecholamines at the tissue level. See Brasch H, Sieroslawski L, Dominiak P. Angiotensin II increases norepinephrine release from atria by acting on angiotensin subtype 1 receptors. Hypertension 1993; 22: 699-704. Furthermore, bradykinin, which is known to release norepinephrine, is not elevated with direct angiotensin II blockade compared with ACE inhibitor therapy. (See Minisi AJ, Thames MD. Distribution of left ventricular sympathetic afferents demonstrated by reflex responses to transmural myocardial ischemia and to intracoronary and epicardial bradykinin. Circulation 1993; 87: 240-246; and Timmermans P, Wong PC, Chiu AT, et al. Angiotensin II receptors and angiotensin II receptor antagonists. Pharmacol Reviews 1993; 45: 205-251.) A small decrease in mean plasma norepinephrine was observed in the present study with losartan compared to captopril. Plasma catecholamine levels may not, however, reflect local cardiac tissue levels, and resting values may not reflect transient increases during stress or ischemia.

In this study, heart failure status appeared to improve to a similar degree in both treatment groups. New York Heart Association functional class improved significantly and to a comparable extent from baseline following long-term treatment with both losartan and captopril (Figure 4). The rate of hospitalization for heart failure in patients treated with losartan was also similar to that observed in patients treated with captopril. Another similarity was the low incidence of death due to progressive heart failure (less than one percent for both losartan and captopril-treated patients), suggesting a similar beneficial effect for both treatments. Overall hospitalizations were however less frequent with losartan treatment versus captoril.

In conclusion, in older patients with symptomatic heart failure, losartan was better tolerated compared to captopril, with fewer patients discontinuing therapy due to adverse effects or being hospitalized, but was not different from captopril in causing persistent renal dysfunction defined as a sustained increase in serum creatinine. The effects on progressive heart failure hospitalization and improvement in New York Heart Association functional class were similar in the two treatment groups. There was, however, a decrease in all-cause mortality observed with losartan compared to captopril treatment, mainly due to a reduction in sudden cardiac death. These findings suggest that treatment with losartan may offer important therapeutic benefits compared to an ACE inhibitor, and may have far reaching implications for the treatment of patients with heart failure.

### EXAMPLE 2

### Exercise Study―Protocols A & B

Protocols A & B were, multicenter, double-blind, randomized, parallel, placebo-controlled studies to investigate the effects of losartan on the exercise capacity and clinical status of patients over the age of 18 with symptomatic heart failure.

Approximately three hundred and fifty (350) patients with symptomatic heart failure (NYHA Functional Class II-IV, at least 50% Class III-IV) and left ventricular ejection fraction ≤40% were randomized to receive either losartan or placebo once daily for 12 weeks in each study. Following a minimum 15-day baseline placebo period, during which a minimum of 5 and maximum of 8 sets of baseline exercise tests (treadmill exercise test and 6-minute walk test) were performed, patients were randomized to treatment with losartan or placebo in a 2:1 ratio, respectively. According to the protocol's, patients must not have received an ACE-inhibitor for at least 6 weeks prior to the third baseline exercise test visit. Treatment with an ACE-inhibitor was not allowed at any time during these studies.

This report summarizes the results of the Meta-analysis performed (as proposed in the Data Analysis Plan) on the observed mortality and congestive heart failure (CHF) hospitalization across these two studies. The mortality and CHF hospitalization were not predefined endpoints in the protocols but it was intended that they be analyzed as a part of safety. Thus results from these analyses should not be interpreted as a test of significance as there were no predefined hypotheses corresponding to these safety parameters. However, these results can simply be regarded as a measure of the disparity between the losartan and placebo groups with respect to the observed results across the two studies. Odds ratio's with 95% confidence intervals have been provided for these variables.

Peto-modified Mantel-Haenszel (Fleiss, J.L., Statistical Methods for Rates and Proportions, Wiley, NY, 1981.) method was used to carry out the Meta-analysis of death and CHF hospitalization rates in the two 12 week exercise studies. This method assumes homogeneity across the studies and provides a test for heterogeneity. Results of the Meta-analysis are displayed in Tables 4-6.

A total of three hundred and fifty one (351) patients were enrolled in the protocol A study; 237 patients were randomized to receive losartan and 114 patients were randomized to receive placebo. There were 4 (3.5%) deaths in the placebo group as compared to 4 (1.7%) in the losartan group during the double-blind therapy or as related to serious adverse events (AE) during double-blind. Additionally there were 10 (8.8%) CHF hospitalizations in the placebo group as compared to 10 (4.2%) in the losartan group during the double-blind therapy or as related to serious AE during double-blind. One patient included in the losartan group discontinued due to worsening HF and was hospitalized 2 days after discontinuation from double-blind period.

Table 4 provides the odds ratio for the Death and CHF hospitalization in the protocol A exercise study. The odds of dying in the placebo group are 2.1 times the odds of dying in the losartan group while the odds of CHF hospitalization in the placebo group are 2.2 times the odds of CHF hospitalization in the losartan group. However, since 1.0 is included in both the 95% confidence intervals the possibility that the odds of dying (or CHF hospitalization) in the placebo group is the same as odds of dying (or CHF hospitalization) in the losartan group can not be excluded (see Table 4).

A total of three hundred and eighty five (385) patients were enrolled in the protocol B exercise study; 254 patients were randomized to receive losartan and 131 patients were randomized to receive placebo. There were 9 (6.9%) deaths in the placebo group as compared to 3 (1.2%) in the losartan group during the double-blind therapy or as related to serious AE during double-blind. Additionally there were 7 (5.3%) CHF hospitalizations in the placebo group as compared to 5 (2.0%) in the losartan group during the double-blind therapy or as related to serious AE during double-blind.

Table 5 provides the odds ratio for the Death and CHF hospitalization in the protocol B exercise study. The odds of dying in the placebo group are 5.6 times the odds of dying in the losartan group while the odds of CHF hospitalization in the placebo group are 2.7 times the odds of CHF hospitalization in the losartan group. The 95% confidence interval for odds ratio of death was (1.61, 19.34). This implies that the odds of dying in the placebo group are greater than the odds of dying in the losartan group. However, since 1.0 is included in the 95% confidence interval for odds ratio of CHF hospitalization, the possibility that the odds of CHF hospitalization in the placebo group is same as the odds of CHF hospitalization in the losartan group can not be excluded (see Table 5).

Since the results from protocols A and B are consistent, a safety Meta-analysis as proposed in the Data Analysis Plan has been performed. Homogeneity between the two studies with respect to odds ratio's for death and CHF hospitalization was found.

Table 6 provides the results of the Meta-analysis of the two 12 week exercise studies. The odds of dying in the placebo group are 3.5 times the odds of dying in the losartan group while the odds of CHF hospitalization in the placebo group are 2.4 times the odds of CHF hospitalization in the losartan group. The 95% confidence intervals for odds ratio's with respect to death and CHF hospitalization are (1.43, 8.77) and (1.19, 4.76), respectively. Thus the odds of dying in the placebo group are significantly greater than the odds of dying in the losartan group. Similarly the odds of CHF hospitalization in the placebo group are significantly greater than the odds of CHF hospitalization in the losartan group.

**TABLE 4**

| CLINICAL EVENTS―PROTOCOL A | | | | | |
|---|---|---|---|---|---|
| | PBO (N=131) | Losartan (N=254) | Odds Ratio | 95% Confidence Intervals | |
| | | | | Lower | Upper |
| Death^{b} | 3.5% | 1.7% | 2.1 | 0.56 | 7.96 |
| CHF Hospitalization (during DB) | 8.8% | 4.2%^{a} | 2.2 | 0.90 | 5.28 |

| | | | | | |
|---|---|---|---|---|---|
| b : during double-blind therapy or was related to SAE during double-blind | | | | | |
| a : one patient discontinued due to worsening HF and was hospitalized 2 days after discontinuation from double-blind. | | | | | |

**TABLE 5**

| CLINICAL EVENTS ― PROTOCOL B | | | | | |
|---|---|---|---|---|---|
| | PBO (N=131) | Losartan (N=254) | Odds Ratio | 95% Confidence Intervals Lower Upper | |
| Death^{b} | 6.9% | 1.2% | 5.6* | 1.61 | 19.34 |
| CHF Hospitalization (during DB) | 5.3% | 2.0% | 2.7 | 0.89 | 8.39 |

| | | | | | |
|---|---|---|---|---|---|
| b: during double-blind therapy or was related to Serious AE during double-blind | | | | | |
| *: odds are greater for placebo group as compared to losartan group | | | | | |

**TABLE 6―**

| Meta-Analysis@ CLINICAL EVENTS―PROTOCOLS A &B | | | | | |
|---|---|---|---|---|---|
| | PBO (N=245) | Losartan (N=491) | Odds Ratio | 95% Confidence Intervals Lower Upper | |
| Death^{b} | 5.3% | 1.4% | 3.5* | 1.43 | 8.77 |
| CHF Hospitalization (during double-blind) | 6.9% | 3.1% ^{a} | 2.4* | 1.19 | 4.76 |

| | | | | | |
|---|---|---|---|---|---|
| @: Peto-modified Mantel-Haenszel method | | | | | |
| b: during double-blind therapy or was related to Serious AE during double-blind | | | | | |
| a: one patient discontinued due to worsening HF and was hospitalized 2 days after discontinuation from double-blind. | | | | | |
| *: odds are greater for placebo group as compared to losartan group | | | | | |

## Claims

1. Use of an angiotensin II (AT₁) receptor antagonist selected from losartan, EXP-3174, or a pharmaceutically acceptable salt thereof, for the manufacture of a medicament for reducing mortality of a symptomatic heart failure patient.

2. Use according to Claim 1 wherein the symptomatic heart failure patient is 65 years of age or older.

3. Use of an angiotensin II (AT₁) receptor antagonist selected from losartan, EXP-3174, or a pharmaceutically acceptable salt thereof, for the manufacture of a medicament for (a) reducing sudden cardiac death of a symptomatic heart patient; (b) reducing mortality and sudden cardiac death of a symptomatic heart failure patient; (c) preventing sudden cardiac death of a symptomatic heart failure patient; or (d) reducing hospitalization of a symptomatic heart failure patient.

4. Use according to Claim 3 wherein the symptomatic heart failure patient is 65 years of age or older.

## Patentansprüche

1. Verwendung eines Angiotensin II (AT₁)-Rezeptor-Antagonisten, ausgewählt aus Losartan, EXP-3174 oder einem pharmazeutisch annehmbaren Salz davon, zur Herstellung eines Medikaments zur Verringerung der Sterblichkeitsrate eines Patienten mit symptomatischer Herzinsuffizienz.

2. Verwendung nach Anspruch 1, wobei der Patient mit symptomatischer Herzinsuffizienz 65 Jahre alt oder älter ist.

3. Verwendung eines Angiotensin II (AT₁)-Rezeptor-Antagonisten, ausgewählt aus Losartan, EXP-3174 oder einem pharmazeutisch annehmbaren Salz davon, zur Herstellung eines Medikaments zur (a) Verringerung von plötzlichem Herztod eines Patienten mit symptomatischer Herzinsuffizienz; (b) Verringerung der Sterblichkeitsrate und von plötzlichem Herztod eines Patienten mit symptomatischer Herzinsuffizienz; (c) Verhinderung von plötzlichem Herztod eines Patienten mit symptomatischer Herzinsuffizienz; oder (d) Verringerung des Krankenhausaufenthalts eines Patienten mit symptomatischer Herzinsuffizienz.

4. Verwendung nach Anspruch 3, wobei der Patient mit symptomatischer Herzinsuffizienz 65 Jahre alt oder älter ist.

## Revendications

1. Utilisation d'un antagoniste des récepteurs de l'angiotensine II (AT₁) choisi parmi le losartan, EXP-3174, ou un de leurs sels pharmaceutiquement acceptables, pour la fabrication d'un médicament destiné à réduire la mortalité d'un patient atteint d'une défaillance cardiaque symptomatique.

2. Utilisation selon la revendication 1, dans laquelle le patient atteint d'une défaillance cardiaque symptomatique est âgé de 65 ans ou plus.

3. Utilisation d'un antagoniste des récepteurs de l'angiotensine II (AT₁) choisi parmi le losartan, EXP-3174, ou un de leurs sels pharmaceutiquement acceptables, pour la fabrication d'un médicament destiné à (a) réduire la mort subite par arrêt cardiaque d'un patient cardiaque symptomatique ; (b) réduire la mortalité et la mort subite par arrêt cardiaque d'un patient atteint d'une défaillance cardiaque symptomatique ; (c) prévenir la mort subite par arrêt cardiaque d'un patient atteint d'une défaillance cardiaque symptomatique; ou (d) réduire l'hospitalisation d'un patient atteint d'une défaillance cardiaque symptomatique.

4. Utilisation selon la revendication 3, dans laquelle le patient atteint d'une défaillance cardiaque symptomatique est âgé de 65 ans ou plus.
